(19)

**Europäisches Patentamt**
**European Patent Office**
**Office européen des brevets**

(11)  **EP 4 494 549 A1**

(12)  **EUROPEAN PATENT APPLICATION**
published in accordance with Art. 153(4) EPC

(43) Date of publication:
**22.01.2025  Bulletin 2025/04**

(21) Application number: **23771051.2**

(22) Date of filing: **13.03.2023**

(51) International Patent Classification (IPC):
*A61B 5/00* (2006.01)       *G16H 10/60* (2018.01)
*G16H 50/30* (2018.01)       *G06N 3/04* (2023.01)

(52) Cooperative Patent Classification (CPC):
**A61B 5/00; G06N 3/04; G16H 10/20; G16H 10/60;**
**G16H 50/30**

(86) International application number:
**PCT/KR2023/003366**

(87) International publication number:
**WO 2023/177172 (21.09.2023 Gazette 2023/38)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **14.03.2022  KR 20220031218**

(71) Applicant: **Gowoonsesang Cosmetics Co., Ltd.**
**Seongnam-si, Gyeonggi-do 13591 (KR)**

(72) Inventor: **AHN, Gun Young**
**Gwangju-si Gyeonggi-do 12773 (KR)**

(74) Representative: **Winter, Brandl - Partnerschaft**
**mbB**
**Alois-Steinecker-Straße 22**
**85354 Freising (DE)**

(54)  **APPARATUS AND METHOD FOR DETERMINING SKIN CONDITION OR SKIN TYPE BY USING ARTIFICIAL NEURAL NETWORK, AND RECORDING MEDIUM HAVING RECORDED THEREON INSTRUCTIONS**

(57)    According to an aspect of the present disclosure, an apparatus for determining a skin condition or skin type of a user based on an artificial intelligence technology may be provided. The apparatus according to the present disclosure may comprise one or more processors; and one or more memories configured to store instructions that, when executed by the processor, cause the one or more processors to perform calculations, and an artificial neural network constructed through modeling of a correlation between a plurality of images for skin of a plurality of users and scores for a plurality of survey questions included in a survey about skin provided to the plurality of users. The one or more processors are configured to, according to the instructions: determine a first survey score group for a survey about skin of a target user; input an image for the skin of the target user into the artificial neural network; determine a second survey score group for the survey, based on an output of the artificial neural network; and determine a score of each of one or more indexes indicating a skin type of the target user or a skin condition of the target user, based on the first survey score group and the second survey score group.

FIG. 1

Computing device (100) — User image (310) and survey result (330) — One or more index scores indicating user skin types and/or user skin conditions — User terminal (110)

**Description**

TECHNICAL FIELD

The present disclosure relates to a technology for determining a skin condition or a skin type of a user based on an artificial intelligence technique.

BACKGROUND

In a method of determining a skin condition or skin type of a user, previously, the skin condition could be determined by the user directly answering a survey or a questionnaire. However, in this method, there are differences depending on the individual's personality in that the person directly determines his/her own condition and there is a problem in that the skin condition cannot be coherently evaluated due to such differences. Further, the skin condition may be conventionally determined by an expert who examines the skin condition of the user based on a user image. This method has a problem in that a skin condition (for example, contact dermatitis or the like) that is difficult to be determined through an image cannot be evaluated. Accordingly, there has been a growing need for a method of more consistently and accurately determining a skin condition in the art.

SUMMARY

The present disclosure provides a technology for determining a skin condition or skin type of a user based on an artificial intelligence technique.

According to an aspect of the present disclosure, an apparatus for determining a skin condition or skin type of a user based on an artificial intelligence technology may be provided. The apparatus according to the present disclosure may comprise one or more processors; and one or more memories configured to store instructions that, when executed by the processor, cause the one or more processors to perform calculations, and an artificial neural network constructed through modeling of a correlation between a plurality of images for skin of a plurality of users and scores for a plurality of survey questions included in a survey about skin provided to the plurality of users, wherein the one or more processors may be configured to, according to the instructions: determine a first survey score group for a survey about skin of a target user; input an image for the skin of the target user into the artificial neural network; determine a second survey score group for the survey, based on an output of the artificial neural network; and determine a score of each of one or more indexes indicating a skin type of the target user or a skin condition of the target user, based on the first survey score group and the second survey score group.

In one embodiment, the survey may comprise question groups for at least one target to be determined, and the question groups for at least one target to be determined may correspond to the one or more indexes, respectively.

In one embodiment, the one or more processors may be configured to: acquire a survey result from the target user, the survey result comprising an answer to each of the plurality of survey questions included in the survey; and determine the first survey score group by converting the answer to each of the plurality of survey questions included in the survey into a score.

In one embodiment, the second survey score group may be determined after the artificial neural network receives an input of the image for the skin of the target user and outputs the score for each of the plurality of survey questions included in the survey.

In one embodiment, the one or more processors may be configured to: acquire a score of a question corresponding to a first type among the plurality of survey questions included in the survey from the first survey score group; acquire a score of a question corresponding to a second type among the plurality of survey questions included in the survey from the second survey score group; determine a final survey score group, based on the score of the question corresponding to the first type and the score of the question corresponding to the second type; and determine a score for the skin type or each of the one or more indexes, based on the final survey score group.

In one embodiment, the question corresponding to the first type may be a question having a score predicted by the artificial neural network, for which accuracy is less than a predetermined threshold value, and the question corresponding to the second type is a question having a score predicted by the artificial neural network, for which accuracy is higher than or equal to the predetermined threshold value.

In one embodiment, the one or more processors may be configured to: determine a final survey score group, based on the first survey score group and the second survey score group; determine the score for each of the one or more indexes by adding up scores of questions included in the final survey score group according to each question group included in the survey; and determine the skin type, based on the score for each of the one or more indexes.

In one embodiment, the one or more processors may be configured to: determine an expert score group for the image for the skin of the target user, based on the output of the artificial neural network; and determine the score for the skin type or each of the one or more indexes, additionally based on the expert score group.

In one embodiment, the expert score group may comprise scores for at least one target to be determined among flush, pigment, pore, wrinkle, and acne determined based on the image for the skin of the target user.

In one embodiment, the one or more processors may be configured to: determine a final survey score group, based on the first survey score group and the second survey score group; and determine the score for the skin type or each of the one or more indexes by calculating a weighted sum of the final survey score group and the expert score group.

According to another aspect of the present disclosure, a method for determining a skin condition or skin type of a user based on an artificial intelligence technology may be provided. A method performed by a computer may comprise one or more processors and one or more memories storing instructions executed by the one or more processors, the one or more memories may store an artificial neural network constructed through modeling of a correlation between a plurality of images for skin of a plurality of users and scores for a plurality of survey questions included in a survey about skin provided to the plurality of users, and the method, by the one or more processors, may comprise, according to the instructions: determining a first survey score group for a survey about skin of a target user; inputting an image for the skin of the target user into the artificial neural network; determining a second survey score group for the survey, based on an output of the artificial neural network; and determining a score of each of one or more indexes indicating a skin type of the target user or a skin condition of the target user, based on the first survey score group and the second survey score group.

In one embodiment, the survey may comprise question groups for at least one target to be determined, and the question groups for at least one target to be determined correspond to the one or more indexes, respectively.

In one embodiment, the method may further comprise, by the one or more processors: acquiring a survey result from the target user, the survey result comprising an answer to each of the plurality of survey questions included in the survey; and determining the first survey score group by converting the answer to each of the plurality of survey questions included in the survey into a score.

In one embodiment, the method may further comprise, by the one or more processors: acquiring a score of a question corresponding to a first type among the plurality of survey questions included in the survey from the first survey score group; acquiring a score of a question corresponding to a second type among the plurality of survey questions included in the survey from the second survey score group; determining a final survey score group, based on the score of the question corresponding to the first type and the score of the question corresponding to the second type; and determining a score for the skin type or each of the one or more indexes, based on the final survey score group.

In one embodiment, the question corresponding to the first type may be a question having a score predicted by the artificial neural network, for which accuracy is less than a predetermined threshold value, and the question corresponding to the second type is a question having a score predicted by the artificial neural network, for which accuracy is higher than or equal to the predetermined threshold value.

In one embodiment, the method may further comprise, by the one or more processors: determining a final survey score group, based on the first survey score group and the second survey score group; determining the score for each of the one or more indexes by adding up scores of questions included in the final survey score group according to each question group included in the survey; and determining the skin type, based on the score for each of the one or more indexes.

In one embodiment, the method may further comprise, by the one or more processors: determining an expert score group for the image for the skin of the target user, based on the output of the artificial neural network; and determining the score for the skin type or each of the one or more indexes, additionally based on the expert score group.

In one embodiment, the expert score group may comprise scores for at least one target to be determined among flush, pigment, pore, wrinkle, and acne determined based on the image for the skin of the target user.

In one embodiment, the method may further comprise, by the one or more processors, determining a final survey score group, based on the first survey score group and the second survey score group, wherein the determining of the score for the skin type or each of the one or more indexes may comprise determining the score for the skin type or each of the one or more indexes by calculating a weighted sum of the final survey score group and the expert score group.

According to another aspect of the present disclosure, a non-transitory computer-readable recording medium for determining a skin condition or skin type of a user based on an artificial intelligence technology may be provided. A non-transitory computer-readable recording medium recording instructions executed by a computer, the non-transitory computer-readable recording medium may comprise one or more memories configured to store instructions that, when executed by the processor, cause the one or more processors to perform calculations, and an artificial neural network constructed through modeling of a correlation between a plurality of images for skin of a plurality of users and scores for a plurality of survey questions included in a survey about skin provided to the plurality of users, wherein the instructions when executed by the one or more processors, may cause the one or more processors to: determine a first survey score group for a survey about skin of a target user; input an image for the skin of the target user into the artificial neural network; determine a second survey score group for the survey, based on an output of the artificial neural network; and determine a score of each of one or more indexes indicating a skin type of the target user or a skin condition of the target user, based on the first survey score group and the second survey score group.

According to various embodiments of the present disclosure, it is possible to provide an explainable artificial neural network, compared to an artificial neural network for predicting a skin condition of a user immediately from a user image, by inputting the user image into the artificial neural network and predicting a score of each survey question included in a survey.

According to various embodiments of the present disclosure, it is possible to improve reliability of the score for the survey by predicting a survey score group (for example, a second survey score group) for the survey through the artificial neural network and correcting a survey score group (for example, a first survey score group) according to real answers of the user by means by the second survey score group.

According to various embodiments of the present disclosure, it is possible to increase user convenience by reducing the number of survey questions to be acquired from the user.

BRIEF DESCRIPTION OF DRAWINGS

FIG. 1 illustrates a process in which a computing device operates according to an embodiment of the present disclosure.
FIG. 2 is a block diagram illustrating the computing device according to an embodiment of the present disclosure.
FIG. 3 is a diagram illustrating a user image and a survey result according to an embodiment of the present disclosure.
FIG. 4 is a conceptual diagram illustrating a process in which the computing device determines a second survey score group based on an output of an artificial neural network according to an embodiment of the present disclosure.
FIG. 5 is a conceptual diagram illustrating a process in which the computing device determines an expert score group and a second survey score group based on the output of the artificial neural network according to another embodiment of the present disclosure.
FIG. 6 illustrates some of training data for training the artificial neural network according to an embodiment of the present disclosure.
FIG. 7 is a flowchart illustrating an operation of the computing device according to an embodiment of the present disclosure.

DETAILED DESCRIPTION

Various embodiments of the present disclosure are examples for the purpose of clearly describing the technical idea of the present disclosure and do not limit the present disclosure to a specific embodiment form. The technical idea of the present disclosure includes various modifications, equivalents, and alternatives of respective embodiments disclosed in this document, and embodiments selectively combined from all or some of the respective embodiments. The scope of the claims of the technical idea of the present disclosure is not limited to various embodiments presented below or detailed description thereof.

The terms used in the present disclosure including technical or scientific terms may have meanings generally understood by those skilled in the art to which the present disclosure belongs unless defined otherwise.

The expressions "include," "may include," "provided with," "may be provided with," "have," "may have" and the like used in the present disclosure indicate the existence of features subject thereto (for example, functions, operations, elements, or the like) and do not exclude the existence of other additional features. That is, the expressions should be understood as openended terms connoting the possibility of inclusion of other embodiments.

A singular expression used in the present disclosure can include meanings of plurality, unless otherwise mentioned in context, and the same is applied to a singular expression recited in the claims.

The expressions "first," "second," and the like used in the present disclosure are used to distinguish one entity from another entity among a plurality of entities, unless otherwise mentioned in context, and are not intended to limit the order or importance of the corresponding entities. In an embodiment, a plurality of question groups according to the present disclosure may be distinguished from each other through the expressions "first question group" and "second question group." In an embodiment, a plurality of indexes indicating skin conditions according to the present disclosure may be distinguished from each other through the expressions "first index" and "second index."

The expressions "A, B, and C," "A, B, or C," "A, B, and/or C," "at least one of A, B, and C," "at least one of A, B, or C," or "at least one of A, B, and/or C" used in the present disclosure may mean each of the listed items or all available combinations of the listed items. For example, "at least one of A or B" may refer to all of (1) at least one A, (2) at least one B, or (3) both of at least one A and at least one B.

The term "unit" used in the present disclosure may mean software or a hardware component such as a field-programmable gate array (FPGA) and an application specific integrated circuit (ASIC). However, the "unit" is not limited to hardware and software. The "unit" may be configured to be stored in an addressable storage medium and may be configured to run one or more processors. In an embodiment, the "unit" may include software components, object-oriented software components, components such as class components and task components, processors, functions, procedures, subroutines, segments of program codes, drivers, firmware, micro-codes, circuits, data, database, data structures, tables, arrays, and parameters.

The expression "based on" used herein is used to describe one or more factors that influences a decision, an action of judgment or an operation described in a phrase or sentence including the relevant expression, and this expression does not exclude additional factor influencing the decision, the action of judgment or the operation.

The expression indicating that any element (for example, a first element) is "connected to" or "coupled to" another element (for example, a second element) used in the present disclosure may not only mean that the any element is directly connected or coupled to the other element but also mean that the any element is connected or coupled thereto via a new element (for example, a third element).

The expression "configured to" used in the present disclosure may have a meaning such as "set to," "having an ability to," "changed to," "made to," "capable of," or the like in context. The corresponding expression is not limited to the meaning "specifically designed in hardware" and, for example, a processor configured to perform a specific operation may mean a special purpose computer structured to perform the corresponding specific operation through programming.

In the present disclosure, artificial intelligence (AI) may mean technology that imitates human learning ability, reasoning ability, and perception ability, and implements the same on a computer. Artificial intelligence may include machine learning and element technology using machine learning. Machine learning may mean an algorithm that extracts characteristics of at least one piece of training data to classify input data. Further, technologies that imitate functions of human brain such as recognition and determination using the algorithm of machine learning may also be understood as a category of artificial intelligence. For example, technical fields such as linguistic understanding, visual understanding, inference/prediction, knowledge representation, and operation control may be included.

In the present disclosure, an artificial neural network may be designed to simulate a human brain structure on a computer and may include a plurality of network nodes which simulate neurons of a human neural network and have weighted values. The plurality of network nodes may simulate synaptic activity of neurons which exchanges signals through synapse between neurons and have a connection relation therebetween. In the artificial neural network, the plurality of network nodes are located on layers having different depths and may exchange data according to a convolution connection relation. The artificial neural network may be, for example, a convolution neural network. In the present disclosure, the artificial neural network is a model trained according to a predetermined machine learning scheme and may mean a model of which a weighted value for at least one network node, which is included in a non-trained model, is determined by machine learning. Machine learning may mean that computer software improves data processing capabilities through training using data and experience of processing the data. The artificial neural network is constructed through modeling of a correlation between data, and the correlation may be expressed by a plurality of parameters. The artificial neural network derives the correlation between data by extracting and analyzing characteristics from the given data, and machine learning may be optimizing parameters of the artificial neural network by repeating the process. For example, the artificial neural network may train mapping (correlation) between the input and the output for data given in the form of a pair of input and output. Alternatively, when only input data is given, the artificial neural network may learn the relation by deriving regularity between the given data. In the present disclosure, the "artificial neural network" may be used interchangeably with the term "artificial neural network model."

Hereinafter, various embodiments of the present disclosure are described with reference to the accompanying drawings. In the accompanying drawings and description of the drawings, the same reference numeral may be assigned to the same or substantially equivalent elements. Further, in the following description of various embodiments, overlapping description of the same or corresponding elements may be omitted, but this does not mean that the corresponding element does not belong to the embodiment.

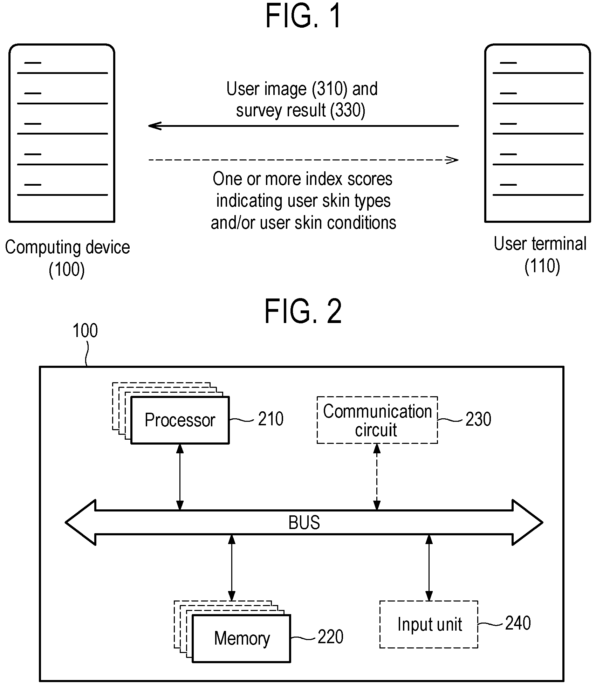

FIG. 1 illustrates a process in which a computing device 100 operates according to an embodiment of the present disclosure. The computing device 100 may acquire a user image 310 and a survey result 330 from a user terminal 110. The user image 310 may be an image for skin of a "target user" who desires to determine a skin type through the computing device 100 or determine scores of one or more indexes indicating a skin type through the computing device 100. Hereinafter, for convenience of description, the "target user" may be briefly referred to as a "user," and the "image for skin of the target user" may be briefly referred to as a "user image." The survey result 330 may be grading data acquired from the user for a survey of the present disclosure. The survey result 330 may be data having a predetermined document structure (for example, a table, a list, a tuple, a dictionary, and the like).

The computing device 100 may determine a score for each of one or more indexes indicating a skin type of the user and/or a skin condition of the user based on the user image 310 and the survey result 330. The computing device 100 may determine a first survey score group for the survey based on the survey result 330. The computing device 100 may input the user image 310 into an artificial neural network and determine a second survey score group for the survey based on an output of the artificial neural network. The computing device 100 may determine a final survey score group based on the first survey score group and the second survey score group. The computing device 100 may determine a score for each of one or more indexes indicating a skin condition based on the final survey score group. The computing device 100 may determine a skin type of the user through the determined score for each of the one or more indexes.

The computing device 100 may transmit the determined skin type and the score for each of the one or more indexes to the user terminal 110.

In the present disclosure, the one or more indexes indicating the skin condition of the user may include indexes such as, for example, oil, sensitive, moisture, pigment, wrinkle, pore, and the like. The computing device 100 may determine the score for each of the one or more indexes indicating the skin condition through predetermined calculations. At this time, a score for a specific index may indicate the quality of the skin condition for the corresponding index. For example, the user's skin lacks oil as the score for the oil index is lower, and the user's skin is excessively oily as the score for the oil index is higher. The user's skin lacks moisture as the score for the moisture index is lower, and the user's skin is in a sufficient moisture condition as the score for the moisture index is higher. The user's skin is not sensitive as the score for the sensitive index is lower, and the user's skin is sensitive as the score for the sensitive index is higher. The user's skin is almost not pigmented as the score for the pigment index is lower, and the user's skin is highly pigmented as the score for the pigment index is higher. The user's skin has no wrinkles as the score for the wrinkle index is lower, and the user's skin has many wrinkles as the score for the wrinkle index is higher. The user's skin is in a good pore condition (for example, the number of pores is small or the size thereof is small) as the score for the pore index is lower, and the user's skin is in a poor pore condition (for example, the number of pores is large or the size thereof is large) as the score for the pore index is higher. The one or more indexes indicating the skin condition is only for description, and the present disclosure includes various types of indexes that can indicate skin conditions.

FIG. 2 is a block diagram of the computing device 100 according to an embodiment of the present disclosure. The computing device 100 may include one or more processors 210 and/or one or more memories 220 as elements. In some embodiments, at least one of the elements of the computing device 100 may be omitted, or other elements may be added to the computing device 100. In some embodiments, additionally or alternatively, some elements may be integrated or may be implemented as a single or a plurality of entities. In the present disclosure, one or more processors 210 may be expressed as a processor 210. The expression "processor 210" may mean a set of one or more processors unless clearly stated otherwise in context. In the disclosure, one or more memories 220 may be expressed as a memory 220. The expression "memory 220" may mean a set of one or more memories unless clearly stated otherwise in context.

At least some elements inside or outside the computing device 100 may be connected to each other through a bus, general purpose input/output (GPIO), a serial peripheral interface (SPI), a mobile industry processor interface (MIPI), or the like and exchange data and/or signals.

The processor 210 may control at least one element of the computing device 100 connected to the processor 210 by driving software (for example, instructions, programs, or the like). The processor 210 may perform an operation of performing various calculations, processing, data generation, manufacturing, and the like related to the present disclosure. The processor 210 may load data and the like from the memory 320 and store the same in the memory 320. The processor 210 may process calculations for an artificial neural network.

The memory 220 may store various pieces of data. Data stored in the memory 220 may be acquired or processed by at least one element of the computing device 100 or may include software (for example, instructions, programs, or the like) as used data. The memory 220 may include volatile memory and/or nonvolatile memory. In the present disclosure, instructions or programs are software stored in the memory 220 and may include an operating system for controlling resources of the computing device 100, an application, and/or middleware for providing various functions to allow the application to use the resources of the computing device 100. The memory 220 may store instructions that when executed by the processor 210 cause the processor 210 to perform calculations. The memory 220 may store a survey provided to the user. The memory 220 may store an artificial neural network constructed through modeling of the correlation between a plurality of images for skin of a plurality of users (that is, a plurality of predetermined users) and scores for a plurality of survey questions included in the survey for skin provided to a plurality of users (that is, a plurality of predetermined users). The survey and the artificial neural network according to the present disclosure are described below in detail.

In an embodiment, the computing device 100 may further include a communication circuit 230. The communication circuit 230 may perform wireless or wired communication between the computing device 100 and a server or between the computing device 100 and other devices. For example, the communication circuit 230 may perform wireless communication according to a scheme such as enhanced mobile broadband (eMBB), ultra reliable low-latency communications (URLLC), massive machine type communications (MMTC), long-tem evolution (LTE), LTE advance (LTE-A), new radio (NR), universal mobile telecommunications system (UMTS), global system for mobile communications (GSM), code division multiple access (CDMA), wideband CDMA (WCDMA), wireless broadband (WiBro), wireless fidelity (Wi-Fi), Bluetooth, near field communications (NFC), global positioning system (GPS), or global navigation satellite system (GNSS). For example, the communication circuit 230 may perform wired communication according to a scheme such as universal serial bus (USB), high definition multimedia interface (HDMI), recommended standard-232 (RS-232), or plain old telephone service (POTS). In an embodiment, the communication circuit 230 may communicate with another device. At such time, the another device may be the user terminal 110.

In an embodiment, the computing device 100 may further include an input unit 240. The input unit 240 may be an element for transferring data physically input from the outside to at least one element included in the computing device 100. For example, the input unit 240 may include a mouse, a keyboard, a touch pad, and the like.

The computing device 100 according to various embodiments of the present disclosure may be a device of various types. For example, the computing device may be a portable communication device, a portable multimedia device, a wearable device, a home appliance, or a device according to a combination of one or more of the above-described devices. The computing device of the present disclosure is not limited to the above-listed devices.

The survey according to an embodiment of the present disclosure may include a plurality of predetermined survey questions. In the present disclosure, the "survey question" may be interchangeably used with a "question." The survey may include a question group for at least one target to be determined. Each question group may include one or more survey questions. The target to be determined may be, for example, oily, sensitive, pigmented, wrinkled, or the like. The survey may include at least one question group among a question group for oily skin, a question group for sensitive skin, a question group for pigmented skin, or a question group for wrinkled skin. For example, the question group for oily skin may include questions as shown in Table 1.

[Table 1]

| Q. What happens to my skin if I don't apply moisturizer? | |
| --- | --- |
| 1 | Always dry |
| 2 | Frequently dry |
| 3 | Sometimes dry |
| 4 | No different than usual |

The question group for sensitive skin may include questions shown in Table 2.

[Table 2]

| Q. I with acne by a dermatologist within the last 5 years. | |
| --- | --- |
| 1 | have never been diagnosed (and my skin is clean) |
| 2 | have never been diagnosed (but I have acne) |
| 3 | have been diagnosed |
| 4 | have been diagnosed (I have severe symptoms) |
| 5 | don't remember if I have been diagnosed |

The question group for pigmented skin may include questions shown in Table 3.

[Table 3]

| Q. I blemishes on my skin. | |
| --- | --- |
| 1 | have never had |
| 2 | have some (but they are gone now) |
| 3 | can see |
| 4 | see a lot of |
| 5 | am not sure if there are any |

The question group for wrinkled skin may include questions shown in Table 4.

[Table 4]

| Q. I usually sunscreen. | |
| --- | --- |
| 1 | don't apply sunscreen at all |
| 2 | occasionally apply sunscreen on days when the sun is strong |

(continued)

| Q. I usually sunscreen. | |
|---|---|
| 3 | apply sunscreen every time I go out |
| 4 | apply sunscreen daily and reapply frequently |

As described above, the content of survey questions included in the survey, the type and number of surveys, the type and number of question groups, and the like are examples for describing the present disclosure and do not limit the present disclosure.

In an embodiment of the present disclosure, each of the question groups included in the survey for at least one target to be determined may correspond to each of the one or more indexes. For example, the question group for "oily skin" may be a question group for determining the skin condition for "oil" among one or more indexes indicating skin conditions of the user. The question group for "sensitive skin" may be a question group for determining the skin condition for "sensitive" among one or more indexes indicating skin conditions of the user.

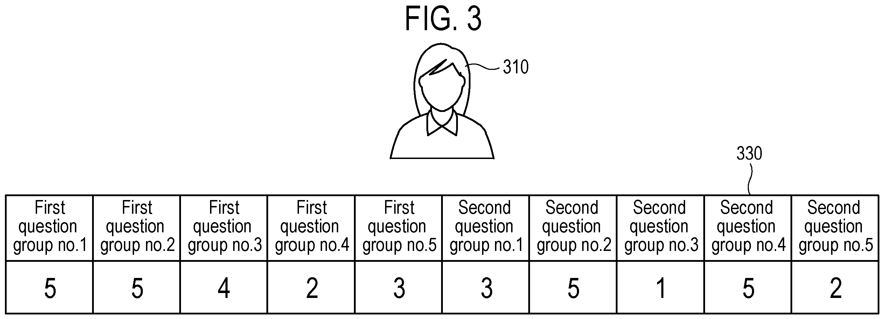

FIG. 3 illustrates a user image 310 and a survey result 330 according to an embodiment of the present disclosure. The user image 310 may be acquired from the user and may be, for example, a facial photo of the user, a full-length photo, or a photo obtained by capturing the user's skin. The survey result 330 may include an answer to each of a plurality of survey questions included in the survey. For example, the survey result 330 may include answers to questions 1 to 5 of a first question group and answers to questions 1 to 5 of a second question group. The computing device 100 may acquire the survey result 330 from the external user terminal 110 through the communication circuit 230. The computing device 100 may acquire the survey result 330 by directly receiving an input signal from the user through the input unit 240. In this case, the computing device 100 may further include a display unit (not shown) for visually showing the survey.

The computing device 100 may determine a first survey score group for the survey based on the survey result 330. In the present disclosure, a survey score group may refer to data including a score for each of a plurality of survey questions included in the survey. A "first survey score group" may refer to a survey score group derived based on the survey result 330 acquired from the user. A "second survey score group" may refer to a survey score group derived based on an output of the artificial neural network. The first survey score group and the second survey score group may be collectively referred to as a "survey score group."

The processor 210 may acquire the survey result 330 including an answer to each of a plurality of survey questions included in the survey from the user. The processor 210 may acquire the survey result 330 by receiving data from an external device (for example, the user terminal) through the communication circuit 230. The processor 210 may acquire the survey result 330 by receiving an input of data from the user through the input unit 240. The processor 210 may determine the first survey score group by converting an answer to each of a plurality of survey questions included in the survey result. Table 5 shows at least some of the first survey score group. The score for each question in Table 5 may be a value obtained by converting the survey result 330 of FIG. 3 into a score.

[Table 5]

| Group | First question group | | | | | Second question group | | | | |
|---|---|---|---|---|---|---|---|---|---|---|
| Questio n | No. 1 | No. 2 | No. 3 | No. 4 | No. 5 | No. 1 | No. 2 | No. 3 | No. 4 | No. 5 |
| Score | 3 | 2.5 | 11 | 4 | 3 | 6 | 2.5 | 1 | 7.5 | 2 |

The survey result may include information on a number selected by the user for a multiple-choice question, and the first survey score group may be data obtained by converting the information on the number selected by the user into the score. A score conversion table used by the processor 210 to convert the survey result 330 into the first survey score group of Table 5 may be, for example, as shown in Table 6.

[Table 6]

| Question group | Question | ① | ② | ③ | ④ | ⑤ |
|---|---|---|---|---|---|---|
| First question group | No. 1 | 1.2 | 2.4 | 3.6 | 4.8 | 3 |
| | No. 2 | 1 | 2 | 3 | 4 | 2.5 |
| | No. 3 | 2.75 | 5.5 | 8.25 | 11 | - |
| | No. 4 | 2 | 4 | 6 | 8 | - |
| | No. 5 | 1 | 2 | 3 | 4 | - |

(continued)

| Question group | Question | ① | ② | ③ | ④ | ⑤ |
|---|---|---|---|---|---|---|
| Second question group | No. 1 | 2 | 4 | 6 | 8 | - |
| | No. 2 | 1 | 2 | 3 | 4 | 2.5 |
| | No. 3 | 1 | 2 | 3 | 4 | - |
| | No. 4 | 3 | 6 | 9 | 12 | 7.5 |
| | No. 5 | 0 | 2 | | | - |

The score conversion table shown in Table 6 may be predetermined in consideration of the number of answers to each question, importance of the answer, and the like. For example, some of the questions included in the survey may be questions for asking about symptoms or gravity of the condition (for example, what happens to my skin if I don't apply moisturizer?). Answers to the question may be configured such that symptoms become severe or relieved from no. 1 to no. 4 (for example, no. 1 always dry, no. 2 frequently dry, no. 3 sometimes dry, and no. 4 no different than usual), and the score conversion table for the corresponding question may be configured to assign a higher score or a less score from no. 1 to no. 4. In another example, some of the questions included in the survey may be questions to which the user cannot know the exact answer or the user has difficulty in answering (for example, I blemishes on my skin). Answers to the question may be configured such that symptoms become severe or relieved from no. 1 to no. 4 but may further include no. 5 corresponding to "don't know," and the score conversion table for the corresponding question may be configured to assign a higher score or a less score from no. 1 to no. 4 but assign a middle score to no. 5. In another example, some of the questions included in the survey may be questions having two choices (for example, true/false, yes/no, or positive/negative). Answers to the question may include two responses, and the score conversion table for the corresponding question may be configured to assign different scores to the two responses.

The computing device 100 may input the user image to the artificial neural network and determine the second survey score group for the survey based on the output of the artificial neural network.

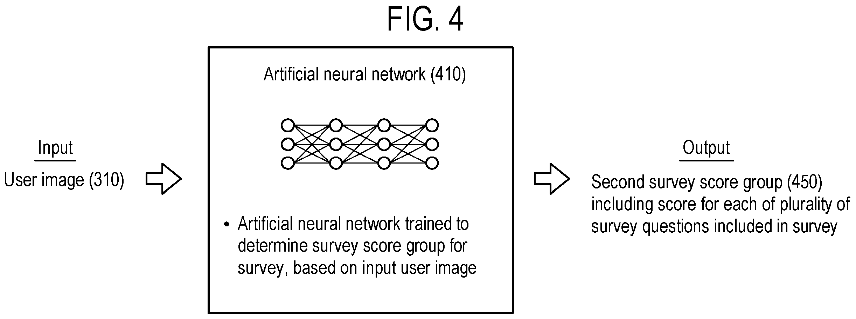

FIG. 4 is a conceptual diagram illustrating a process in which the computing device 100 determines a second survey score group based on an artificial neural network 410 according to an embodiment of the present disclosure. The artificial neural network 410 may be an artificial neural network model trained to determine a second survey score group 450 for the survey based on the input user image 310. The artificial neural network 410 may be trained by the computing device 100 of the present disclosure. The artificial neural network 410 may be transmitted to the computing device 100 and used by the computing device 100 after the artificial neural network 410 is trained by an external device. Hereinafter, it is described that the artificial neural network 410 is trained by the computing device 100, but it is only for convenience of description and does not limit the present disclosure.

The artificial neural network 410 according to an embodiment of the present disclosure may be trained based on one or more pieces of training data including training images and survey score groups determined for the training images based on the survey. The training image is an image pre-received from a predetermined user and may be image stored in the memory 220. For example, the training image included in training data may be an image which is the same as or similar to the user image 310 illustrated in FIG. 3. The survey score group determined for the training image may be data generated based on the survey result responding to the survey by the predetermined user shown in the corresponding training image. The survey score group determined for the training image may be data generated based on the survey result responding to the survey by another user (for example, a skin expert) based on the corresponding training image. Each piece of the training data may be expressed by an ordered pair of X (input value) and Y (label value). X is a training image and Y may be expressed as a survey score group. That is, the training data may be an ordered pair of (training image, survey score group). The artificial neural network 410 may be trained based on the training data.

The processor 210 may input the user image 310 into the trained artificial neural network 410, perform calculations based on at least one node, and determine a second survey score group 450 for the survey including a plurality of survey questions. That is, the second survey score group 450 may be determined after the trained artificial neural network 410 receives the user image 310 and outputs a score for each of the plurality of survey questions included in the survey. Table 7 shows at least some of the second survey score groups. The score for each question shown in Table 7 may be a value output by the artificial neural network 410 based on the user image 310 of FIG. 3.

[Table 7]

| Group | First question group | | | | | Second question group | | | | |
|---|---|---|---|---|---|---|---|---|---|---|
| Questio n | No. 1 | No. 2 | No. 3 | No. 4 | No. 5 | No. 1 | No. 2 | No. 3 | No. 4 | No. 5 |
| Score | 2.37 | 1 | 5 | 3.993 | 1.12 | 6.003 | 2.513 | 2 | 3 | 1.765 |

According to various embodiments of the present disclosure in which the processor 210 determines the second survey score group 450 through the artificial neural network 410, the present disclosure has an advantage of providing an explainable artificial neural network compared to the artificial neural network for predicting a skin condition of a user immediately from a user image by predicting a score of each survey question for the user image 310. Further, the present disclosure predicts the survey score group (that is, the second survey group) for the survey through the artificial neural network, thereby improving reliability of the score for the survey by correcting the survey score group (that is, the first survey score group) according to an actual user response by the second survey score group. In addition, when the survey score group for the survey is predicted using the artificial neural network according to the present disclosure, it is possible to increase user convenience by reducing the number of survey questions that should be acquired from the user.

The computing device 100 may determine a score for each of one or more indexes indicating a skin type of the user or a skin condition of the user based on the first survey score group and the second survey score group. The computing device 100 may determine a final survey score group based on the first survey score group and the second survey score group. The computing device 100 may determine a score for each of one or more indexes indicating a skin condition based on the final survey score group. The computing device 100 may determine a skin type of the user through the determined score for each of the one or more indexes. Hereinafter, various embodiments in which the computing device 100 according to the present disclosure determines the final survey score group based on the first survey score group and the second survey score group are described.

In a first embodiment in which the computing device 100 determines a final survey score group, the processor 210 may determine the final survey score group by adding up the first survey score group and the second survey score group or deriving an average value. The processor 210 may determine the final survey score group by adding up or averaging scores included in the first survey score group determined based on the survey result acquired from the user and the second survey score group determined based on the output of the artificial neural network 410 for the user image. Table 8 shows the final survey score group calculated by averaging the first survey score group shown in Table 5 and the second survey score group shown in Table 7.

[Table 8]

| Group | First question group | | | | | Second question group | | | | |
|---|---|---|---|---|---|---|---|---|---|---|
| Quest ion | No. 1 | No. 2 | No. 3 | No. 4 | No. 5 | No. 1 | No. 2 | No. 3 | No. 4 | No. 5 |
| First score | 3 | 2.5 | 11 | 4 | 3 | 6 | 2.5 | 1 | 7.5 | 2 |
| Secon d score | 2.37 | 1 | 5 | 3.993 | 1.12 | 6.003 | 2.513 | 2 | 3 | 1.765 |
| Final score | 2.685 | 1.75 | 8 | 3.997 | 2.06 | 6.002 | 2.507 | 1.5 | 5.25 | 1.883 |

In a second embodiment in which the computing device 100 determines the final survey score group, the processor 210 may determine the final survey score group by calculating a weighted average for the first survey score group and the second survey score group. A weighted value of each survey score group for the weighted average may be a value predetermined for each of the first survey score group and the second survey score group. For example, the weighted value "0.3" may be determined for the first survey score group, and the weighted value "0.7" may be determined for the second survey score group. The weighted value for each survey score group may be determined in consideration of accuracy of the artificial neural network 410. The weighted value of each survey score group for the weighted average may be based on reliability of the artificial neural network 410. Reliability which the artificial neural network 410 outputs together with the second survey score group for the input image may be, for example, a real number within a range [0, 1]. The processor 210 may calculate the weighted average for the first survey score group and the second survey score group by assigning a higher weighted value to the second survey score group as the reliability output by the artificial neural network 410 is higher. For example, when the reliability output by the artificial neural network 410 is "P (P being a real number higher than or equal to 0 and equal to or less than 1)", the processor 210 may determine the final survey score group based on an equation of "(1-P) * (first survey score group) + (second survey score group)." Accordingly, as the artificial neural network 410 has higher certainty for the output second survey score group, the processor 210 may reflect more of the second survey score group in the final survey score group. Further, the artificial neural network 410 may output reliability for each question to output the second survey score group. When the artificial neural network 410 derives reliability for each question, calculating the weighted average of the first survey score group and the second survey score group may be performed for each question. Table 9 shows the final survey score group calculated by assigning a weighted value 0.3 to the first survey score group shown in Table 5 and a weighted value 0.7 to the second survey score group shown in Table 7.

[Table 9]

| Grou p | First question group | | | | | Second question group | | | | |
|---|---|---|---|---|---|---|---|---|---|---|
| Quest ion | No. 1 | No. 2 | No. 3 | No. 4 | No. 5 | No. 1 | No. 2 | No. 3 | No. 4 | No. 5 |
| First score | 3 | 2.5 | 11 | 4 | 3 | 6 | 2.5 | 1 | 7.5 | 2 |
| Secon d score | 2.37 | 1 | 5 | 3.993 | 1.12 | 6.003 | 2.513 | 2 | 3 | 1.765 |
| Final score | 2.559 | 1.45 | 6.8 | 3.995 | 1.68 | 6.002 | 2.509 | 1.7 | 4.35 | 1.836 |

In a third embodiment in which the computing device 100 determines the final survey score group, the processor 210 may determine the final survey score group by acquiring scores of the survey questions according to each question from the first survey score group or the second survey score group. Specifically, the processor 210 may divide a plurality of survey questions included in the survey into two or more types and acquire the score of a question corresponding to a first type from the first survey score group and the score of a question corresponding to a second type from the second survey score group. In the present disclosure, the question corresponding to the first type (for example, a first type question) may be a question, for which the score included in the final survey score group is acquired from the first survey score group. Further, in the present disclosure, the question corresponding to the second type (for example, a second type question) may be a question, for which the score included in the final survey score group is acquired from the second survey score group. The processor 210 may determine whether each question corresponds to the first type or the second type based on a predetermined calculation.

In an embodiment of determining whether each of a plurality of questions included in the survey corresponds to the first type or the second type, the processor 210 may determine a type of each question according to a predetermined reference. The predetermined reference may be stored in the memory 220 and may include numbers of questions corresponding to the first type and numbers of questions corresponding to the second type. For example, the survey may include question nos. 1 to no. 10, and the processor 210 may determine question nos. 1 to no. 5 as the first type and question nos. 6 to no. 10 as the second type with reference to the predetermined reference. Further, the processor 210 may acquire scores of questions no. 1 to no. 5 from the first survey score group and scores of question nos. 6 to no. 10 from the second survey score group based on the determined type, so as to determine the final survey score groups for all questions.

In another embodiment of determining whether each of the plurality of questions included in the survey corresponds to the first type or the second type, the processor 210 may determine a type of each question according to accuracy of the artificial neural network 410. The question corresponding to the first type may be a question, for which accuracy of the artificial neural network 410 for predicting the score of the question is less than a predetermined threshold value. The question corresponding to the second type may be a question, for which accuracy of the artificial neural network 410 for predicting the score of the question is higher than or equal to the predetermined threshold value. The accuracy of the artificial neural network 410 according to an embodiment of the present disclosure may be measured during a validation or test process performed after training of the artificial neural model. The accuracy of the artificial neural network 410 may be calculated for each question. For convenience of description, it is assumed that there is a verification data set and the verification data set includes 10 pairs of data. The data pair included in the verification data set may have the form of a tuple (user image, first survey score group). Further, for convenience of description, it is assumed that the verification data set includes (image A, survey score group 1-A (first survey score group corresponding to image A)). Based on such assumption, the artificial neural network 410 may receive an input of the image A and output a survey score group 2-A (second survey score group output for image A). The processor 210 may calculate accuracy of the artificial neural network 410 for image A based on an error in the survey score group 2-A and the survey score group 1-A. For example, the processor 210 may obtain accuracy of the artificial neural network 410 by calculating a relative error in consideration of survey score group 1-A of "true value" and survey score group 2-A of "measurement value." For example, when the survey score group 1-A made based on the survey including two questions and the image A is [3, 4] and the output survey score group 2-A is [2.4, 3.6], accuracy of question no. 1 may be calculated as 0.8 (= 1 - |3 - 2.4|/3) and accuracy of question no. 2 may be calculated as 0.9 (= 1 - |4 - 3.6|/4). Further, when such a process is performed for all 10 pairs of data included in the verification data set, the processor 210 may calculate accuracy of the verification data set of the artificial neural network 410. For example, the processor 210 may calculate accuracy of the verification data set by averaging the accuracy calculated for each piece of verification data. As a result, the processor 210 may determine accuracy of the artificial neural network 410. The processor 210 according to the present disclosure may determine each of a plurality of questions as the first type or the second type by comparing the accuracy of the artificial neural network 410 calculated for each question with a predetermined threshold value. For example, when accuracy of questions of the artificial neural network 410 calculated for the survey including question no. 1 and question no. 2 are 0.8 and 0.9 and the predetermined threshold value is 0.85, the processor 210 may determine question no. 1 having accuracy less than the predetermined threshold value as the first type and determine question no. 2 having accuracy higher than the predetermined threshold value as the second type. As a result, the processor 210 may acquire the score of question no. 1 from the first survey score group and acquire the score of question no. 2 from the second survey score group, so as to determine the final survey score group for both question no. 1 and question no. 2. The predetermined threshold value to be compared with the accuracy of the artificial neural network 410 calculated for each question may be configured for each question. When the threshold value is configured for each question, the processor 210 may determine a type of the question in consideration of different threshold values for respective questions.

The processor 210 may classify each of the plurality of questions included in the survey as one of the first type or the second type based on at least one method described above and acquire the score of the corresponding question from the first survey score group or the second survey score group according to the type of the question, so as to determine the final survey score group. For the first survey score group shown in Table 5 and the second survey score group shown in Table 7, Table 10 shows the final survey score group determined according to the type of each question. For convenience of description, it is assumed that question nos. 2, 3, and 5 of the first question group and question nos. 3 and 4 of the second question group are first type questions and question nos. 1 and 4 of the first question group and question nos. 1, 2, and 5 of the second question group are second type questions.

[Table 10]

| Group | First question group | | | | | Second question group | | | | |
|---|---|---|---|---|---|---|---|---|---|---|
| Questio n | No. 1 | No. 2 | No. 3 | No. 4 | No. 5 | No. 1 | No. 2 | No. 3 | No. 4 | No. 5 |
| First score | 3 | 2.5 | 11 | 4 | 3 | 6 | 2.5 | 1 | 7.5 | 2 |
| Second score | 2.37 | 1 | 5 | 3.993 | 1.12 | 6.003 | 2.513 | 2 | 3 | 1.765 |
| Final score | 2.37 | 2.5 | 11 | 3.993 | 3 | 6.003 | 2.513 | 1 | 7.5 | 1.765 |

As described above, some embodiments for determining the final survey score group described with reference to Table 8 to Table 10 and some embodiments for determining types of questions are only examples for describing the present disclosure and do not limit the present disclosure, and the present disclosure includes various embodiments for determining the final survey score group based on the first survey score group determined based on the user input and the second survey score group determined based on the output of the artificial neural network.

The computing device 100 may determine the score of each of one or more indexes indicating a user skin type or the user skin condition based on the final survey score group. In the present disclosure, the score for each of the one or more indexes indicating user skin conditions may include a "total score" and an "index score." The processor 210 may determine the total score by adding up scores of questions included in the final survey score group according to at least one question group included in the survey. For example, with reference to the final survey score group of Table 10, the total score of the first question group may be 22.863 and the total score of the second question group may be 18.781. When the first question group is a question group for determining an oily skin and the second question group is a question group for determining a sensitivity degree, the processor 210 may determine the total score for an oil index as 22.863 and the total score for a sensitivity index as 18.781. The processor 210 may determine the index score for each of one or more indexes indicating a user skin condition based on the total score. The processor 210 may convert the total score into an index score through a scaling scheme. In the present disclosure, the scaling scheme may refer to a scheme for converting a score range of the determined total score into a predetermined score range (for example, higher than or equal to 0 and equal to or less than 100). For example, the processor 210 may perform calculations based on Equation 1 for scaling.

[Equation 1]

$$Index\ Score(Converted\ Score) = (Total\ Score * (\tfrac{100}{10})) - ((\tfrac{100}{10}) * 4)$$

For example, when the "total score" calculated for a specific index by the processor 210 is 13 and the processor 210 performs the scaling scheme according to Equation 1, the "index score" for the corresponding specific index may be 90. According to the present disclosure, the processor 210 may determine which skin condition corresponds to the corresponding index score within a predetermined total score range by converting a score range that the specific index can have (that is, [minimum total value of scores for theoretically available questions, a maximum total value of scores for theoretically available questions] into a predetermined score range (for example, [0, 100]).

The processor 210 may determine the user skin condition indicated by the corresponding index based on the index score for each of the one or more indexes. For example, when a first range (for example, higher than or equal to 0 and less than 20) is classified as "very bad," a second range (for example, higher than or equal to 20 and less than 40) is classified as "bad," a third range (for example, higher than or equal to 40 and less than 60) is classified as "normal," a fourth range (for example, higher than or equal to 60 and less than 80) is classified as "good," and a fifth range (for example, higher than or equal to 80 and equal to or less than 100) is classified as "very good" in the total score range (for example, higher than or equal to 0 and equal to or less than 100) predetermined for the specific index, and the index score for the specific index is 55, the processor 210 may determine the user skin condition indicated by the corresponding specific index as "normal."

The processor 210 may determine the user skin type based on the user skin condition for each of the one or more indexes. For example, it is assumed that a first index included in the one or more indexes is an oil index, a second index is a moisture index, and a third index is a sensitivity index. The processor 210 may determine a skin condition for each index by comparing an index score for each index with an average score of the corresponding index. For example, in the oil index corresponding to the first index, an oil condition of the user may be determined as "dry (D)" when an oil index score of the user is less than an average score for the oil index and determined as "oily (O)" when the oil index score of the user is higher than the average score for the oil index. In the moisture index corresponding to the second index, a moisture condition of the user may be determined as "moisture deficit (-)" when a moisture index score of the user is less than an average score for the moisture index and determined as "sufficient moisture (+)" when the moisture index score of the user is higher than the average score for the moisture index. A sensitivity condition of the user may be determined as "sensitive (S)" when a sensitivity index score of the user is less than an average score for the sensitivity index and determined as "non-sensitive (N)" when the sensitivity index score of the user is higher than the average score for the sensitivity index. The processor 210 may determine a user skin type by adding up skin conditions according to the determined indexes. That is, when the oil condition determined for the user (that is, the skin condition according to the first index) is "dry (D)," the moisture condition (that is, the skin condition according to the second index) is "moisture deficit (-)," and the sensitivity condition (that is, the skin condition according to the third index) is "sensitive (S)," the processor 210 may determine the corresponding user skin type as "DS-." In this case, "DS-" may be a skin type collectively referring to dry, moisture deficit, and sensitive skin. As described above, the processor 210 may determine skin conditions for respective indexes based on the index score for each of the one or more indexes and combine the determined skin conditions, so as to determine the user skin type. For example, when there are six indexes indicating the skin condition and each index has two conditions, the user skin type may include a total of 64 (=$2^6$) types. The number of conditions for each index may be three or more. In another embodiment, the processor 210 may determine a user skin type based on a main index included in one or more indexes. For example, when one or more indexes indicating the skin condition includes first to sixth indexes, the processor 210 may determine a user skin type by combining only skin conditions for one or more indexes (for example, first to third indexes) corresponding to main indexes. For example, when the user skin type is determined based on three main indexes among the six indexes indicating the skin condition, a total of 8 (=$2^3$) types may be derived as the user skin type. In this case, the processor 210 according to the present disclosure has an advantage of transmitting the user skin type in the form of shorter and more intuitive information and generating additional information through index scores or skin conditions determined for indexes which are not the main indexes.

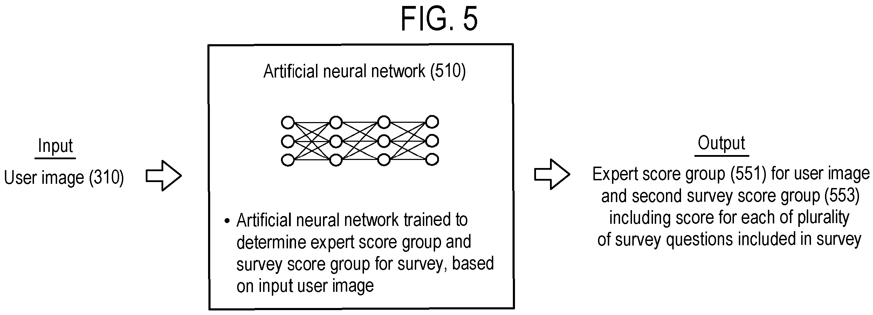

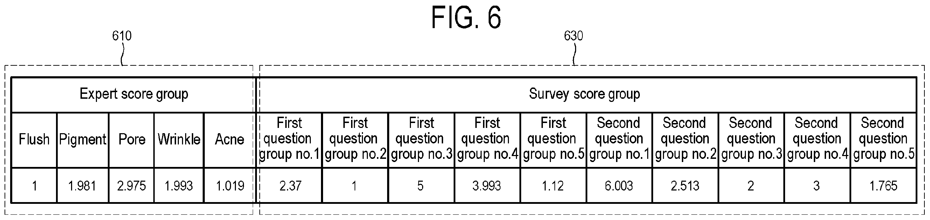

Hereinafter, input and output data and operations of the artificial neural network 510 according to another embodiment of the present disclosure are described with reference to FIGS. 5 and 6. In the present disclosure, unless expressed otherwise, the above description of the artificial neural network 510 may be applied to the artificial neural network 510 and overlapping description is omitted hereinafter.

FIG. 5 is a conceptual diagram illustrating a process in which the computing device 100 determines an expert score group 551 and a second survey score group 553 based on an output of the artificial neural network 510 according to another embodiment of the present disclosure.

The computing device 100 may determine the expert score group 551 for the user image 310 based on the output of the artificial neural network 510. That is, the computing device 100 may determine the second survey score group for the survey based on the output of the artificial neural network 510 and additionally determine the expert score group 551 for the user image 310. The expert score group 551 may include scores for one or more targets to be determined, which can be visually determined based on the user image 310. The expert score group 551 may include scores for at least one target to be determined among flush, pigment, pore, wrinkle, and acne. For example, the artificial neural network 510 may determine the expert score group 551 by determining a flush score as 1, a pigment score as 1.981, a pore score as 2.975, a wrinkle score as 1.993, and an inflammation score as 1.019 based on the user image 310.

FIG. 6 illustrates some of training data for training the artificial neural network 510 according to an embodiment of the present disclosure. The artificial neural network 510 may be trained based on one or more pieces of data including a survey score group 630 determined for a training image based on the survey and an expert score group 610 determined for the training image. When the artificial neural network 610 is trained by the training data, the artificial neural network 510 may determine the expert score group 551 and the second survey score group 553 based on the input user image 310. The training image included in the training data and configurations and/or a generation method of the survey score group 630 have been described above with reference to FIGS. 3 and 4. The expert score group 610 included in the training data may be data generated as a result of viewing the corresponding training image with the naked eye by an expert (for example, a health care provider, a skin-related expert, or the like). For example, the expert score group 610 included in the training data may include scores determined for at least one target to be determined among flush, pigment, pore, wrinkle, and acne after the expert views the training image with the naked eye. When training data of the artificial neural network 510 is expressed by an ordered pair of X (input value) and Y (label value), X may be a training image and Y may be a value including the expert score group 610 and the survey score group 630. That is, the training data may be an ordered pair of (X: training image, Y: [expert score group, survey score group]). In the embodiment, Y included in the training data of the artificial neural network 510 may be data in the form of concatenating the expert score group 610 and the survey score group 630. For example, when the survey score group 630 is a matrix having a size of 1x35 and the expert score group is a matrix having a size of 1x5, Y included in the training data of the artificial neural network 510 may be matrix data having a size of 1x40. The artificial neural network 510 may be trained based on the training data.

The processor 210 may input the user image 310 into the trained artificial neural network 510, perform calculations based on at least one node, and determine the expert score group 551 for the user image 310 and the second survey score group 553 for the survey.

The processor 210 may determine the score for each of the one or more indexes indicating the user skin type or the user skin condition based on the final survey score group and additionally the expert score group determined for the user image based on the artificial neural network 510. The processor 210 may determine the score of the user skin type or each of the one or more indexes by calculating a weighed sum of the final survey score group and the expert score group. For example, when the score for the first index included in the final survey score group is "5," the score for the first index included in the expert score group is "15," and weighted values for the final survey score group and the expert score group are "0.5" and "0.5," the final score for the first index may be determined as "10 (=0.5*5 + 0.5*15)."

The computing device 100 according to the present disclosure may determine the second survey score group 553 and the expert score group 551 based on the artificial neural network 510 and determine the score for the user skin condition and the skin type in consideration of both the survey score group 553 and the expert score group 551, so as to generate skin-related information by combining subjective answers of the user to the survey and objective evaluation of the expert.

In another embodiment of the present disclosure, the computing device 100 may detect at least one target symptom area in the user image 310 based on the artificial neural network. Hereinafter, for convenience of description, the artificial neural network for detecting the target symptom area may be referred to as a "second artificial neural network." The second artificial neural network may be trained through the computing device 100 of the present disclosure. The second artificial neural network may be transmitted to the computing device 100 and used by the computing device 100 after being trained by an external device. The second artificial neural network may receive an image to be trained with for detecting the target symptom area. The target symptom area may be a symptom area such as, for example, acne, pigment, flush, pore, or wrinkle. For example, the second artificial neural network may generate bounding box data including a target symptom area within an image input according to a predetermined detection method. In another example, the second artificial neural network may determine one or more pixels corresponding to the target symptom area among a plurality of pixels included in an image input according to a predetermined segmentation method. The processor 210 may detect at least one target symptom area within the user image 310 through the second artificial neural network which has been completely trained and display the detected area through a display unit.

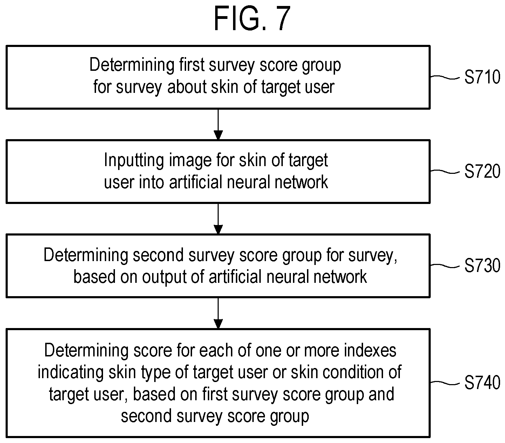

FIG. 7 is a flowchart illustrating an operation of the computing device according to an embodiment of the present disclosure. The computing device 100 according to an embodiment of the present disclosure may determine a first survey score group for the survey about skin of a target user in operation S710. The target user may refer to a user who desires to determine a skin type through the computing device 100 or determine scores for one or more indexes indicating skin conditions through the computing device 100. The survey may include a plurality of predetermined survey questions. The survey may include a question group for at least one target to be determined. The survey may include at least one question group among a question group for oily skin, a question group for sensitive skin, a question group for pigmented skin, or a question group for wrinkled skin. Each question group included in the survey may include a plurality of survey questions. The processor 210 may acquire the survey result as the answer of the user to the survey. The processor 210 may acquire the survey result 330 from the user through the input unit 240. The processor 210 may acquire the survey result 330 from the external user terminal 110 through the communication unit 230. The processor 210 may determine a first survey score group for the survey based on the acquired survey result 330. A "survey score group" may refer to data including a score for each of a plurality of survey questions included in the survey. The "first survey score group" may refer to a survey score group derived based on the survey result 330 acquired from the user. For example, the survey result 330 may include an answer (for example, a response number) selected for each objective question, and the first survey score group may be data obtained by converting the survey result 330 into the score. The processor 210 may determine the first survey score group by converting an answer to each of a plurality of survey questions included in the survey result.

The computing device 100 according to an embodiment of the present disclosure may input an image for skin of the target user into the artificial neural network in operation S720. The artificial neural network may be trained to determine the survey score group for the survey based on the input user image. Training data for training the artificial neural network may include a training image and a survey score group for the corresponding training image.

The computing device 100 according to an embodiment of the present disclosure may determine a second survey score group for the survey based on an output of the artificial neural network in operation S730. The "second survey score group" may refer to a survey score group derived based on an output of the artificial neural network.

The computing device 100 according to an embodiment of the present disclosure may determine scores for one or more indexes indicating a skin type of the target user or a skin condition of the target user based on the first survey score group and the second survey score group in operation S740. The processor 210 may determine a final survey score group by calculating a sum, an average, or a weighted sum of the first survey score group and the second survey score group. The processor 210 may determine the final survey score group by determining scores for a plurality of survey questions included in the survey from one of the first survey score group or the second survey score group. In this case, a question type may be determined according to a predetermined reference in the memory 220. Further, the question type may be determined based on accuracy of the artificial neural network for each question. The processor 210 may determine the score for each of the one or more indexes indicating a user skin type or a user skin condition based on the final survey score group. In the present disclosure, the score for each of the one or more indexes indicating the user skin condition may include a "total score" and an "index score." The processor 210 may determine the total score by summing scores of questions included in the final survey score group according to at least one question group included in the survey. The processor 210 may determine the index score for each of one or more indexes indicating the user skin condition based on the total score. The processor 210 may convert the total score into an index score through a scaling scheme. The processor 210 may determine the user skin condition indicated by the corresponding index based on the index score for each of the one or more indexes. The processor 210 may determine the user skin type based on the user skin condition for each of the one or more indexes.

Although respective steps of a method or an algorithm according to the present disclosure in each flowchart in the present disclosure are described in a sequential order, the respective steps may be performed in an order randomly combined by the present disclosure as well as in the sequential order. The description according to the flowchart does not exclude changes or modifications in the method or the algorithm and does not mean that a predetermined step is necessary or preferable. In an embodiment, at least some operations may be performed in parallel, repeatedly, or heuristically. In an embodiment, at least some operations may be omitted or other operations may be added.

Various embodiments of the present disclosure may be implemented as software in a machine-readable storage medium. The software may be software for implementing various embodiments of the present disclosure. The software may be inferred from various embodiments of the present disclosure by programmers in the art to which the present disclosure belongs. For example, the software may be a program including machine-readable instructions (for example, codes or code segments). The machine may be a device which can operate according to instructions called from a storage medium and may be, for example, a computer. In an embodiment, the machine may be the computing device 100 according to embodiments of the present disclosure. In an embodiment, a processor of the machine may allow elements of the machine to perform functions corresponding to the corresponding instructions by executing the called instructions. In an embodiment, the processor may be the processor 210 according to embodiments of the present disclosure. The storage medium may be all types of recording medium storing machine-readable data. The storage medium may include, for example, a ROM, a RAM, a CD-ROM, a magnetic tape, a floppy disk, an optical data storage device, and the like. In an embodiment, the storage medium may be the memory 320. In an embodiment, the storage medium may be implemented in a form distributed to computer systems which are connected through a network. The software may be distributed to and stored in the computer systems and executed. The storage medium may be a non-transitory storage medium. The non-transitory storage medium is a tangible medium regardless of semi-permanently or temporarily stored data and does not include a transitorily spread signal.

Although the technical idea of the present disclosure has been described by various embodiments, the technical idea of the present disclosure includes various replacements, modifications, and changes which can be made within the scope that can be understood by those skilled in the art to which the present disclosure pertains. Further, the replacements, modifications, and changes should be understood as being included in the scope of the accompanying claims.

**Claims**

1. An apparatus comprising:

   one or more processors; and
   one or more memories configured to store instructions that, when executed by the processor, cause the one or more processors to perform calculations, and an artificial neural network constructed through modeling of a correlation between a plurality of images for skin of a plurality of users and scores for a plurality of survey questions included in a survey about skin provided to the plurality of users,
   wherein the one or more processors are configured to, according to the instructions:

   determine a first survey score group for a survey about skin of a target user;
   input an image for the skin of the target user into the artificial neural network;
   determine a second survey score group for the survey, based on an output of the artificial neural network; and
   determine a score of each of one or more indexes indicating a skin type of the target user or a skin condition of the target user, based on the first survey score group and the second survey score group.

2. The apparatus of claim 1, wherein the survey comprises question groups for at least one target to be determined, and wherein the question groups for at least one target to be determined corresponds to the one or more indexes, respectively.

3. The apparatus of claim 1, wherein the one or more processors are configured to:

   acquire a survey result from the target user, the survey result comprising an answer to each of the plurality of survey questions included in the survey; and
   determine the first survey score group by converting the answer to each of the plurality of survey questions included in the survey into a score.

4. The apparatus of claim 1, wherein the second survey score group is determined after the artificial neural network receives an input of the image for the skin of the target user and outputs the score for each of the plurality of survey questions included in the survey.

5. The apparatus of claim 1, wherein the one or more processors are configured to:

   acquire a score of a question corresponding to a first type among the plurality of survey questions included in the survey from the first survey score group;
   acquire a score of a question corresponding to a second type among the plurality of survey questions included in the survey from the second survey score group;
   determine a final survey score group, based on the score of the question corresponding to the first type and the score of the question corresponding to the second type; and
   determine a score for the skin type or each of the one or more indexes, based on the final survey score group.

6. The apparatus of claim 5, wherein the question corresponding to the first type is a question having a score predicted by the artificial neural network, for which accuracy is less than a predetermined threshold value, and the question corresponding to the second type is a question having a score predicted by the artificial neural network, for which accuracy is higher than or equal to the predetermined threshold value.

7. The apparatus of claim 1, wherein the one or more processors are configured to:

   determine a final survey score group, based on the first survey score group and the second survey score group;
   determine the score for each of the one or more indexes by adding up scores of questions included in the final survey score group according to each question group included in the survey; and
   determine the skin type, based on the score for each of the one or more indexes.

8. The apparatus of claim 1, wherein the one or more processors are configured to:

   determine an expert score group for the image for the skin of the target user, based on the output of the artificial neural network; and

determine the score for the skin type or each of the one or more indexes, additionally based on the expert score group.

9. The apparatus of claim 8, wherein the expert score group comprises scores for at least one target to be determined among flush, pigment, pore, wrinkle, and acne determined based on the image for the skin of the target user.

10. The apparatus of claim 8, wherein the one or more processors are configured to:

   determine a final survey score group, based on the first survey score group and the second survey score group; and
   determine the score for the skin type or each of the one or more indexes by calculating a weighted sum of the final survey score group and the expert score group.

11. A method performed by a computer comprising one or more processors and one or more memories storing instructions executed by the one or more processors,

   the one or more memories storing an artificial neural network constructed through modeling of a correlation between a plurality of images for skin of a plurality of users and scores for a plurality of survey questions included in a survey about skin provided to the plurality of users, and
   the method, by the one or more processors, comprising, according to the instructions:

      determining a first survey score group for a survey about skin of a target user;
      inputting an image for the skin of the target user into the artificial neural network;
      determining a second survey score group for the survey, based on an output of the artificial neural network; and
      determining a score of each of one or more indexes indicating a skin type of the target user or a skin condition of the target user, based on the first survey score group and the second survey score group.

12. The method of claim 11, wherein the survey comprises question groups for at least one target to be determined, and the question groups for at least one target to be determined correspond to the one or more indexes, respectively.

13. The method of claim 11, further comprising, by the one or more processors:

   acquiring a survey result from the target user, the survey result comprising an answer to each of the plurality of survey questions included in the survey; and
   determining the first survey score group by converting the answer to each of the plurality of survey questions included in the survey into a score.

14. The method of claim 11, further comprising, by the one or more processors:

   acquiring a score of a question corresponding to a first type among the plurality of survey questions included in the survey from the first survey score group;
   acquiring a score of a question corresponding to a second type among the plurality of survey questions included in the survey from the second survey score group;
   determining a final survey score group, based on the score of the question corresponding to the first type and the score of the question corresponding to the second type; and
   determining a score for the skin type or each of the one or more indexes, based on the final survey score group.

15. The method of claim 14, wherein the question corresponding to the first type is a question having a score predicted by the artificial neural network, of which accuracy is less than a predetermined threshold value, and the question corresponding to the second type is a question having a score predicted by the artificial neural network, of which accuracy is higher than or equal to the predetermined threshold value.

16. The method of claim 11, further comprising, by the one or more processors:

   determining a final survey score group, based on the first survey score group and the second survey score group;
   determining the score for each of the one or more indexes by adding up scores of questions included in the final survey score group according to each question group included in the survey; and

determining the skin type, based on the score for each of the one or more indexes.

17. The method of claim 11, further comprising, by the one or more processors:

determining an expert score group for the image for the skin of the target user, based on the output of the artificial neural network; and
determining the score for the skin type or each of the one or more indexes, additionally based on the expert score group.

18. The method of claim 17, wherein the expert score group comprises scores for at least one target to be determined among flush, pigment, pore, wrinkle, and acne determined based on the image for the skin of the target user.

19. The method of claim 17, further comprising, by the one or more processors, determining a final survey score group, based on the first survey score group and the second survey score group,
wherein the determining of the score for the skin type or each of the one or more indexes comprises determining the score for the skin type or each of the one or more indexes by calculating a weighted sum of the final survey score group and the expert score group.

20. A non-transitory computer-readable recording medium recording instructions executed by a computer, the non-transitory computer-readable recording medium comprising one or more memories configured to store instructions that, when executed by the processor, cause the one or more processors to perform calculations, and an artificial neural network constructed through modeling of a correlation between a plurality of images for skin of a plurality of users and scores for a plurality of survey questions included in a survey about skin provided to the plurality of users, wherein the instructions, when executed by the one or more processors, cause the one or more processors to:

determine a first survey score group for a survey about skin of a target user;
input an image for the skin of the target user into the artificial neural network;
determine a second survey score group for the survey, based on an output of the artificial neural network; and
determine a score of each of one or more indexes indicating a skin type of the target user or a skin condition of the target user, based on the first survey score group and the second survey score group.

EP 4 494 549 A1

# FIG. 1

User image (310) and
survey result (330)

One or more index scores
indicating user skin types
and/or user skin conditions

Computing device
(100)

User terminal
(110)

# FIG. 2

100

Processor — 210

Communication
circuit — 230

BUS

Memory — 220

Input unit — 240

## FIG. 3

| First question group no.1 | First question group no.2 | First question group no.3 | First question group no.4 | First question group no.5 | Second question group no.1 | Second question group no.2 | Second question group no.3 | Second question group no.4 | Second question group no.5 |
|---|---|---|---|---|---|---|---|---|---|
| 5 | 5 | 4 | 2 | 3 | 3 | 5 | 1 | 5 | 2 |

# FIG. 4

Input
_____
User image (310) ⇨

Artificial neural network (410)

- Artificial neural network trained to determine survey score group for survey, based on input user image

⇨ Output
_____
Second survey score group (450) including score for each of plurality of survey questions included in survey

EP 4 494 549 A1

# FIG. 5

**Input**

User image (310) ⇨

**Artificial neural network (510)**

- Artificial neural network trained to determine expert score group and survey score group for survey, based on input user image

⇨ **Output**

Expert score group (551) for user image and second survey score group (553) including score for each of plurality of survey questions included in survey

EP 4 494 549 A1

# FIG. 6

610

630

| Expert score group | | | | | Survey score group | | | | | | | | | |
|---|---|---|---|---|---|---|---|---|---|---|---|---|---|---|
| Flush | Pigment | Pore | Wrinkle | Acne | First question group no.1 | First question group no.2 | First question group no.3 | First question group no.4 | First question group no.5 | Second question group no.1 | Second question group no.2 | Second question group no.3 | Second question group no.4 | Second question group no.5 |
| 1 | 1.981 | 2.975 | 1.993 | 1.019 | 2.37 | 1 | 5 | 3.993 | 1.12 | 6.003 | 2.513 | 2 | 3 | 1.765 |

# FIG. 7

| Determining first survey score group for survey about skin of target user | S710 |

↓

| Inputting image for skin of target user into artificial neural network | S720 |

↓

| Determining second survey score group for survey, based on output of artificial neural network | S730 |

↓

| Determining score for each of one or more indexes indicating skin type of target user or skin condition of target user, based on first survey score group and second survey score group | S740 |

<table>
<tr><td colspan="2" align="center">**INTERNATIONAL SEARCH REPORT**</td><td>International application No.<br>**PCT/KR2023/003366**</td></tr>
</table>

**A. CLASSIFICATION OF SUBJECT MATTER**

**A61B 5/00**(2006.01)i; **G16H 10/60**(2018.01)i; **G16H 50/30**(2018.01)i; **G06N 3/04**(2006.01)i

According to International Patent Classification (IPC) or to both national classification and IPC

**B. FIELDS SEARCHED**

Minimum documentation searched (classification system followed by classification symbols)

A61B 5/00(2006.01); A45D 44/00(2006.01); G06N 3/02(2006.01); G06Q 30/06(2012.01); G16H 50/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 피부(skin), 이미지(image), 설문지(questionnaire), 점수(score), 인공 신경망(artificial neural network)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2022-0013781 A (NCYC CO., LTD.) 04 February 2022 (2022-02-04)<br>See paragraphs [0056], [0058], [0065] and [0071]; and claims 1-3. | 1-20 |
| Y | KR 10-2021-0079229 A (JEJU TECHNOPARK) 29 June 2021 (2021-06-29)<br>See claim 2. | 1-20 |
| Y | KR 10-2020-0030138 A (BIZMODELINE CO., LTD.) 20 March 2020 (2020-03-20)<br>See paragraph [0097]; and claim 1. | 8-10,17-19 |
| A | KR 10-2020-0028571 A (LAPARTORY) 17 March 2020 (2020-03-17)<br>See paragraphs [0064]-[0160]; and figures 1-2. | 1-20 |
| A | KR 10-2021-0122560 A (AI2U) 12 October 2021 (2021-10-12)<br>See paragraphs [0040]-[0048]; and figure 2. | 1-20 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **22 June 2023** | **22 June 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/003366**

| Patent document cited in search report | | Publication date (day/month/year) | Patent family member(s) | | | Publication date (day/month/year) |
|---|---|---|---|---|---|---|
| KR 10-2022-0013781 | A | 04 February 2022 | KR | 10-2402684 | B1 | 27 May 2022 |
| KR 10-2021-0079229 | A | 29 June 2021 | None | | | |
| KR 10-2020-0030138 | A | 20 March 2020 | None | | | |
| KR 10-2020-0028571 | A | 17 March 2020 | None | | | |
| KR 10-2021-0122560 | A | 12 October 2021 | WO | 2021-201582 | A1 | 07 October 2021 |

Form PCT/ISA/210 (patent family annex) (July 2022)